# EUROPÄISCHE PATENTSCHRIFT

(11) **EP 0 405 120 B1**
(45) Veröffentlichungstag und Bekanntmachung des Hinweises auf die Patenterteilung: **14.04.1993**
(21) Anmeldenummer: 90109395.5
(22) Anmeldetag: 18.05.1990
(51) Int. Cl.: A61K 7/09

(54) **Mittel zur dauerhaften Verformung von Haaren**
Composition for permanent deformation of hair
Composition pour la déformation permanente des cheveux

(30) Priorität: 27.06.1989 DE 3920984
(43) Veröffentlichungstag der Anmeldung: 02.01.1991
(73) Patentinhaber: GOLDWELL AKTIENGESELLSCHAFT, D-64297 Darmstadt (DE)
(72) Erfinder: Tennigkeit, Jürgen, Dr. Dipl.-Ing., D-6104 Seeheim 2 (DE); Rose, Burkhard, Chemie-Ing. (grad), D-6100 Darmstadt 13 (DE); Dornbusch, Klaus, Dr., D-8771 Erlenbach (DE)

(56) Entgegenhaltungen:
- DE-A- 3 608 151
- DE-A- 3 707 415
- US-A- 4 313 933
- "HANDBUCH DER KOSMETIKA UND RIECHSTOFFE", III. Band: "DIE K RPERPFLEGEMITTEL", 1973 H.JANISTYN, Dr. Alfred HUthig Verlag, Heidelberg Seiten 359-363

## Beschreibung

Die Erfindung betrifft ein Mittel zur dauerhaften Verformung von Haaren auf der Basis eines Thioglykolsäureesters als Reduktionsmittel, vorzugsweise eines Glycerinmonothioglykolsäureesters sowie die Anwendung des Mittels zur dauerhaften Verformung von Haaren.

Gegenüber den früher ausschließlich verwendeten mildalkalischen Dauerwell- oder Dauerverformungsmitteln auf Thioglykolatbasis sind in den letzten Jahren schwach saure bis neutral eingestellte Dauerverformungsmittel auf der Basis eines Thioglykolsäureesters entwickelt worden, welche eine deutlich geringere Schädigung der Struktur von empfindlichen oder durch vorausgehende Dauerwell- oder Färbebehandlung vorgeschädigten Haaren bewirken. Insoweit haben sich Verformungsmittel auf der Basis eines Thioglykolsäureesters insbesondere für die Verformungsbehandlung von vorgeschädigten Haaren bewährt. Obwohl diese "Esterwellen" im allgemeinen bei vorschriftsmäßiger Anwendung von den behandelten Personen gut vertragen werden, kommt es vor, daß besonders disponierte Personen sensibilisiert werden.

Der Erfindung liegt die Aufgabe zugrunde, ein Dauerverformungsmittel auf Esterbasis anzugeben, das im Vergleich zu den bekannten Dauerverformungsmittel auf der Basis von Thioglykolsäureestern bei mindesetens gleicher Umformungswirksamkeit und Schonung der Haarstruktur eine deutlich geringere Neigung zu Sensibilisierungen zeigt.

Ausgehend von einem Verformungsmittel der eingangs erwähnten Art wird diese Aufgabe erfindungsgemäß dadurch gelöst, daß das Dauerverformungsmittel neben dem Thioglykolsäureester einen Anteil von 2-Mercaptopropionsäuremonoglycerinester und/oder 3-Mercaptopropionsäuremonoglycerinester als Verformungsmittel und wenigstens einen Lösungsvermittler enthält.

Es wurde gefunden, daß bei einem Anteil des 2-Mercaptopropionsäuremonoglycerinesters und/oder des 3-Mercaptopropionsäuremonoglycerinesters bezogen auf ein ausschließlich einen Thioglykolsäureester als verformungswirksamen Bestandteil enthaltendes Dauerverformungsmittel im Bereich von 10 bis 60% des letzteren eine wesentliche Verringerung des Sensibilisierungspotentials erreicht wird, wobei ein besonders bevorzugter Bereich bei einem Anteil von 20 bis 30% des 2-Mercaptopropionsäuremonoglycerinesters und/oder des 3-Mercaptopropionsäuremonoglycerinesters liegt.

Das erfindungsgemäße Dauerverformungsmittel wird dabei in üblicher Weise so angewandt, daß das Haar bevor und/oder nachdem es - z.B. durch Aufwickeln auf Wickler - in eine gewünschte Form gebracht wird bzw. gebracht wurde, mit dem Dauerverformungsmittel behandelt, dann mit Wasser gespült und anschließend zur Fixierung der Dauerverformung oxidativ nachbehandelt und schließlich getrocknet wird.

Die Einwirkungszeit des Verformungsmittels kann dabei zwischen 5 min. bis 60 min. liegen.

In üblicher Weise kann während der Einwirkung des Verformungsmittels auch zusätzlich Wärme auf das Haar zur Einwirkung gebracht werden, wobei der Einwirkungszeitraum dann im Bereich zwischen 5 min. und 40 min. liegt.

Vergleichsversuche bezüglich der Wellwirksamkeit des erfindungsgemäßen Verformungsmittels im Vergleich zu den bekannten Verformungsmitteln auf der Basis eines Thioglykolsäureesters ergaben, daß das modifizierte Verformungsmittel eine wenigstens gleiche - tendenziell sogar geringfügig stärkere - Wellwirkung wie das unmodifizierte Produkt zeigt, ohne daß stärkere Schädigungen der Haarstruktur der umgeformten Haare beobachtet werden. Dies gilt sowohl für die Modifizierung des Dauerverformungsmittels mit 2-Mercaptopropionsäuremonoglycerinester als auch mit 3-Mercaptopropionsäuremonoglycerinester oder mit Gemischen der beiden Ester.

Zur Abschätzung des Sensibilisierungspotentials des neuen Haarverformungsmittels im Vergleich zu den üblichen "Sauer-Dauerwellen" wurden drei, sich nur in der "Reduktionsphase" unterscheidende Rezepturen A, B und C unter gleichen Bedingungen mit dem Sensibilisierungstest gemäß den OECD-Guidelines for Testing of Chemicals, section 4: Number 406, "Skin Sensitization" vom 12.5.1981 (entsprechend: Directive 84/449, EEC B.6. "Acute Toxicity - Skin Sensitization") und "Allergic Contact Dermatitis in the Guinea-Pig: Identification of Contact Allgerns" Magnusson B. Kligman A.M., 1970 published by C.C. Thomas, Springfield, Illinois, USA, untersucht. Die Reduktionsphase wurden bei diesen Rezepturen gebildet bei A von handelsüblichem Glycerinmonothioglykolsäureester (GMTG) ca. 75%-ig in Glycerin, bei B von 70% handelsüblichem (GMTG) (wie bei A), 25% 2-Mercaptopropionsäuremonoglycerinester ca.80%-ig in Glycerin und 5% Lösungsvermittler, während bei C gegenüber der Rezeptur B anstelle des 2-Mercaptopropionsäuremonoglycerinesters die gleiche Menge 3-Mercaptopropionsäuremonoglycerinester verwendet wurde. Als Ergebnis ist festzuhalten,daß die modifizierten Rezepturen B und C im Vergleich zu der nach den Kriterien der angewandten Tests als "mäßig sensibilisierend" einzustufenden konventionellen Rezeptur A praktisch keine Sensibilisierung zur Folge hatten.

Zusammengefaßt zeigt sich also, daß die modifizierten Haarverformungsmittel praktisch kein Sensibilisierungspotential aufweisen, ohne daß hierfür eine Verringerung der Wellwirksamkeit oder eine stärkere Schädigung der Haarstruktur in Kauf genommen werden muß.

Als Lösungsvermittler kommen in Frage Fettsäurepolyethylenglykolester, Nonylphenolpolyglykoläther und ähnliche in der Kosmetik übliche Lösungsvermittler.

Aus der US-A 4 313 933 ist eine Reduktionslösung zum Dauerwellen von Haaren bekannt, die auf einen pH-Wert eingestellt ist, der um 0,1 bis 0,5 Einheiten niedriger als der isoelektrische Punkt des Haares liegt.

Als Reduktionsmittel können, wie üblich Thioglykolsäure, Thiomilchsäure und deren Gemische eingesetzt werden. Ester dieser Säuren sind hingegen nicht erwähnt.

Die DE-A 36 08 151 beschreibt die Verwendung von Dipropylenglykolmonomethylether als Quellungs- und Penetrationsmittel in Haarverformungsmitteln. Es können auch Mercaptocarbonsäureester als wirksame Reduktionsmittel verwendet werden, als solche sind Monothioglykolsäureglykolester und -glycerinester genannt. Auch Thiomilchsäure und deren Ammonium- oder Guanidinsalze können für diesen Zweck eingesetzt werden.

Die erfindungsgemäße Kombination läßt sich daraus nicht ableiten.

## Patentansprüche

1. Mittel zur dauerhaften Verformung von Haaren auf der Basis eines Thioglykolsäureesters als Reduktionsmittel, vorzugsweise eines Glycerinmonothioglykolsäureesters,
**dadurch gekennzeichnet,**
daß das Dauerverformungsmittel neben dem Thioglykolsäureester einen Anteil von 2-Mercaptopropionsäuremonoglycerinester und/oder 3-Mercaptopropionsäuremonoglycerinester als Verformungsmittel und wenigstens einen Lösungsvermittler enthält.

2. Mittel nach Anspruch 1, dadurch gekennzeichnet, daß der Anteil des 2-Mercaptopropionsäuremonoglycerinesters und/oder des 3-Mercaptopropionsäuremonoglycerinesters bezogen auf ein ausschließlich einen Thioglykolsäureester als verformungswirksamen Bestandteil enthaltendes Dauerverformungsmittel im Bereich von 10 bis 60% des Reduktionsmittels liegt.

3. Mittel nach Anspruch 2, dadurch gekennzeichnet, daß der Anteil des 2-Mercaptopropionsäuremonoglycerinesters und/oder des 3-Mercaptopropionsäuremonoglycerinesters im Bereich zwischen 20 bis 30% liegt.

## Claims

1. Composition for permanent waving of hair based on a thioglycolic ester as reducing agent, preferably a glycerol monothioglycolic ester, characterized in that the permanent waving composition contains 2-mercaptopropionic monoglycerol ester and (or) 3-mercaptoperopionic monoglycerol ester as waving agent in addition to the thioglycolic acid ester, and at least one solubilizer.

2. Composition according to claim 1, characterized in that the percentage of the 2-mercaptopropionic acid monoglycerol ester and (or) of the 3-mercaptopropionic acid monoglycerol ester is from 10 to 60% of the reducing agent, calculated to a permanent shaving composition based exclusively on a thioglycolic ester.

3. Composition according to claim 2, characterized in that the percentage of 2-mercaptopropionic acid monoglycerol ester and (or) 3-mercaptopropionic acid monoglycerol ester is in the range from 20 to 30%.

## Revendications

1. Composition pour la déformation permanente des cheveux, à base d'un ester de l'acide thioglycolique comme réducteur,avantageusement d'un monoester glycérolique d'acide thioglycolique ,caractérisée en ce que la composition pour réaliser une déformation permanente contient, en plus de l'ester d'acide thioglycolique,une certaine proportion de monoester glycérolique d'acide 2-mercaptopropionique et/ou de monoester glycérolique d'acide 3-mercaptopropionique comme agent de déformation et au moins un adjuvant de solubilité .

2. Composition selon la revendication 1 , caractérisée en ce que la proportion du monoester glycérolique d'acide 2-mercaptopropionique et/ou du monoester glycérolique d'acide 3-mercaptopropionique se situe, par rapport à une composition pour déformation permanente contenant exclusivement un ester d'acide thioglycolique comme constituant à rôle de déformation, entre 10 et 60 % du réducteur .

3. Composition selon la revendication 2 , caractérisée en ce que la proportion du monoester glycérolique de l'acide 2-mercaptopropionique et/ou du monoester glycérolique de l'acide 3-mercaptopropionique se situe entre 20 et 30 %
